# EUROPEAN PATENT APPLICATION

(11) **EP 4 717 250 A2**
(43) Date of publication of application: **01.04.2026**
(21) Application number: 25203148.9
(22) Date of filing: 18.09.2025
(51) Int. Cl.: A61K 6/17, A61C 13/083, A61K 6/833, A61K 6/836, A61K 6/20

(54) **DENTAL PORCELAIN COMPOSITION AND MANUFACTURING METHOD THEREOF**

(30) Priority: 27.09.2024 JP 2024169692
(71) Applicant: Shofu Inc., Kyoto-shi, Kyoto 605-0983 (JP)
(72) Inventor: TANAKA, Yosuke, Kyoto-shi, 605-0983 (JP); GOTO, Masanori, Kyoto-shi, 605-0983 (JP)
(74) Representative: Eisenführ Speiser

(57) **Abstract**

To provide a dental porcelain composition that can be used for a dental prosthesis device, particularly a dental prosthesis device made of ceramics, glass-ceramics, or glass, does not require skilled techniques, suppresses dripping during technical work and a sink mark after firing, and exhibits excellent aesthetic property after firing and a manufacturing method thereof. To provide a dental porcelain composition comprising (A) glass and (B) organic solvent having a boiling point of 100 to 300°C, wherein the (A) glass includes (A-1) base material glass having a particle diameter D50 of 2 to 8 µm and (A-2) base material glass having a particle diameter D50 of 25 to 45 µm, and a particle diameter of the glass (A) satisfies D10 ≤ 5 µm, 5 µm < D50 < 28 µm, and 28 µm ≤ D90.

## Description

### BACKGROUND OF THE INVENTION

### Field of the Invention

The present invention relates to a dental porcelain composition and a manufacturing method thereof, which can be used for a dental prosthesis device, particularly a dental prosthesis device made of ceramics, glass ceramics or glass.

### Description of the Related Art

As materials for preparing a dental prosthesis device such as an inlay, a crown and a bridge, there is a growing demand for core materials that are less allergenic and have excellent strength and aesthetic property, such as oxide ceramics such as alumina and zirconia, and glass ceramics such as feldspar-base glass and lithium silicate glass. In recent years, with the development of CAD/CAM technology, it has become possible to easily manufacture a shape of a dental prosthesis device from these core materials.

However, considering the actual fit for a patient, there are cases where a slight addition of a porcelain layer (add-on) is necessary to a form of an adjacent tooth surface of a dental prosthesis device or to a pontic portion of a bridge which are manufactured in a dental laboratory. Such minor modifications are performed not only at a dental laboratory but also at a dental clinic, and are a time-sensitive technical work.

Japanese Unexamined Patent Application Publication No. 2017-193492 discloses a dental porcelain powder as a material for partial modifications to a dental prosthesis device made of ceramics or glass-ceramics. The feature is that porcelain powder is slurryed with a kneading liquid, and a porcelain layer is added by repeatedly condensing and absorbing water, and firing.

Japanese Unexamined Patent Application Publication No. 2001-079019 discloses a dental porcelain paste that is hardly dried and solidied during use. The feature is that 100 parts by weight of a paste is formed by mixing 7 to 45 parts by weight of an organic solvent having a viscosity of 50,000 to 1,500,000 cps and dissolved with polymeric material, and a base material glass powder having an average particle diameter of 10 µm. By applying and firing the porcelain paste, it is possible to burn off an organic component to add only a porcelain layer. Therefore, it is possible to eliminates the need for operations like condensation and water absorption to simplify the technical work.

### SUMMARY OF THE INVENTION

### Technical Problem

However, there has been a problem in the prior art disclosed in Japanese Unexamined Patent Application Publication No. 2017-193492 and Japanese Unexamined Patent Application Publication No. 2001-079019. The method, described in Japanese Unexamined Patent Application Publication No. 2017-193492, in which condensation and water absorption are repeatedly perform by slurrying porcelain powder with a mixing liquid, requires skilled technique, and is not a desirable method, particularly for partial and restorative work in dental clinics where speed is required. Furthermore, in the case of a porcelain paste that does not require skilled techniques, as described in Japanese Unexamined Patent Application Publication No. 2001-079019, organic components which have not removed exist until firing begins. Therefore, in this case, paste dripping during technical work and a sink mark such as peeling or crack in the porcelain layer after firing occur.

The problem to be solved by the present invention is to provide a dental porcelain composition that can be used for a dental prosthesis device, particularly a dental prosthesis device made of ceramics, glass-ceramics, or glass, does not require skilled techniques, suppresses dripping during technical work and a sink mark after firing, and exhibits excellent aesthetic property after firing and a manufacturing method thereof.

### Solution to Problem

As a result of intensive studies to solve the above problem, the present inventors have found that the above problem can be solved by combining two kinds of base material glasses having specific particle diameter distributions and adding an organic solvent having a specific boiling point. The present invention encompasses the following content.

The present invention provides a dental porcelain composition comprising (A) glass and (B) organic solvent having a boiling point of 100 to 300 °C, wherein
the (A) glass includes (A-1) base material glass having a particle diameter D50 of 2 to 8 µm and (A-2) base material glass having a particle diameter D50 of 25 to 45 µm, and
a particle diameter of the glass (A) satisfies D10 ≤ 5 µm, 5 µm < D50 < 28 µm, and 28 µm ≤ D90.

The present invention provides manufacturing method of a dental porcelain composition, wherein
a dental porcelain composition is manufactured by mixing (A-1) base material glass having a particle diameter D50 of 2 to 8 µm, (A-2) base material glass having a particle diameter D50 of 25 to 45 µm, and (B) organic solvent having a boiling point of 100 to 300 °C, and
a particle diameter of the glass (A) including the (A-1) base material glass and the (A-2) base material glass satisfies D10 ≤ 5 µm, 5 µm < D50 < 28 µm, and 28 µm ≤ D90.

The present invention provides a manufacturing method of a dental prosthesis device, comprising
a step of applying the dental porcelain composition according to claim 1 to a core material, and
a step of firing the applied dental porcelain composition.

The present invention provides a kit of dental porcelain composition for manufacturing a dental porcelain composition, comprising
(A-1) base material glass having a particle diameter D50 of 2 to 8 µm,
(A-2) base material glass having a particle diameter D50 of 25 to 45 µm, and
(B) organic solvent having a boiling point of 100 to 300 °C.

### Advantageous Effects of Invention

The present invention may provide a dental porcelain composition that can be used for a dental prosthesis device, particularly a dental prosthesis device made of ceramics, glass-ceramics, or glass, does not require skilled techniques, suppresses dripping during technical work and a sink mark after firing, and exhibits excellent aesthetic property after firing and a manufacturing method thereof. The present invention also provides a dental porcelain composition that exhibits excellent dripping resistance during technical work and excellent aesthetic property after firing and a manufacturing method thereof.

### Brief description of drawings

[FIG. 1] Exterior view showing the shape of the zirconia base material used in the test.
[FIG. 2] Front view showing the shape of the zirconia base material used in the test.
[FIG. 3] Cross-sectional view showing the shape of the zirconia base material used in the test.

### DETAILED DESCRIPTION OF THE PREFERRED EMBODIMENTS

The dental porcelain composition according to the present invention comprises (A) glass and (B) organic solvent having a boiling point of 100 to 300 °C, wherein the (A) glass includes (A-1) base material glass having a particle diameter D50 of 2 to 8 µm and (A-2) base material glass having a particle diameter D50 of 25 to 45 µm, and a particle diameter of the glass (A) satisfies D10 ≤ 5 µm, 5 µm < D50 < 28 µm, and 28 µm ≤ D90.

In the present invention, a mixing ratio of the (A-1) base material glass and the (A-2) base material glass may be 9:1 to 1:9 by mass ratio.

In the present invention, a value of (D90-D10)/D50 for the particle diameter of the glass (A) may be 2.8 or more.

In the present invention, softening points according to ISO 6872:2015/Amd.1:2018 "Dentistry-Ceramic materials" of the (A-1) base material glass and the (A-2) base material glass may be 650 °C or less.

In the present invention, a difference in softening point according to ISO 6872:2015/Amd.1:2018 "Dentistry-Ceramic materials" between the base material glass (A-1) and the base material glass (A-2) may be less than 100 °C.

In the present invention, a compounding amount of the (B) organic solvent may be 10 to 200 parts by mass with respect to 100 parts by mass of the (A) glass.

In the present invention, the (B) organic solvent may consist of an organic solvent having a boiling point of 250 °C or less.

In the present invention, the (A) glass may consists of the (A-1) base material glass and the (A-2) base material glass..

In the method for manufacturing a dental porcelain composition according to the present invention, a dental porcelain composition is manufactured by mixing (A-1) base material glass having a particle diameter D50 of 2 to 8 µm, (A-2) base material glass having a particle diameter D50 of 25 to 45 µm, and (B) organic solvent having a boiling point of 100 to 300 °C, and a particle diameter of the glass (A) including the (A-1) base material glass and the (A-2) base material glass satisfies D10 ≤ 5 µm, 5 µm < D50 < 28 µm, and 28 µm ≤ D90.

The method for manufacturing a dental porcelain composition according to the present invention may comprise a step of mixing the (A-1) base material glass and the (A-2) base material glass to manufacture a powder, and a step of kneading the (B) organic solvent with the powder.

The method for manufacturing a dental porcelain composition according to the present invention may comprise a step of kneading the (A-1) base material glass and the (B) organic solvent to manufacture a paste, and a step of mixing the (A-2) base material glass with the paste.

The method for manufacturing a dental prosthesis device according to the present invention may comprise a step of applying the dental porcelain composition according to claim 1 to a core material, and a step of firing the applied dental porcelain composition.

In the present invention, the core material may be made of ceramics, glass ceramics or glass.

The kit of dental porcelain composition for manufacturing a dental porcelain composition according to the present invention comprises (A-1) base material glass having a particle diameter D50 of 2 to 8 µm, (A-2) base material glass having a particle diameter D50 of 25 to 45 µm, and (B) organic solvent having a boiling point of 100 to 300 °C.

### [Dental porcelain composition]

The glass layer obtained by firing the dental porcelain composition of the present invention once or multiple times is not particularly limited, but may be built up with a layer thickness of 1.0 mm or less, preferably 0.8 mm or less and more preferably 0.5 mm or less with respect to a core material. The "core material" in the present invention means a material constituting a dental prosthesis device, particularly a dental prosthesis device made of ceramics, glass ceramics or glass. Examples include oxide ceramics such as alumina and zirconia, and glass or glass ceramics such as feldspar-base glass and lithium silicate glass. The "layer thickness" of the glass layer in the present invention means the numerical value of the elevation of the porcelain layer surface after firing with respect to the core material surface before firing, and can be easily measured using a micrometer, a caliper, a dental thickness gauge, or similar tool, with the surface of the surface core material before firing as the reference plane.

As the temperature for the firing a dental porcelain composition (hereinafter referred to as firing temperature), the temperature range of 650 to 1000°C is generally selected. When the firing is performed at a temperature lower than 650 °C, there is a case where the organic solvent applied simultaneously is not fired sufficiently and is carbonized, therefore it is impossible to obtain the desired color tone. Furthermore, when the firing is performed at a temperature higher than 1000 °C, there is a case where deformation of the core material itself and dripping of the dental porcelain composition occur. In particular, in the dental ceramic composition of the present invention, it is possible to perform firing at 650 °C to 850 °C and it is possible to apply to a core material which is prepared from a lithium disilicate base glass ceramics which includes a risk of deformation in firing more than 850 °C.

The dental porcelain composition of the present invention may be fired only once. Further, firing can be performed multiple times, such as by firing after building up the dental porcelain composition, and then by firing after further building up the dental porcelain composition. Such firing can be performed by using a dental technique porcelain furnace capable of vacuum firing at a heating rate of 10 to 100 °C/min and a firing temperature range of 100 to 1200 °C comprising a drying mechanism outside the furnace.

### [(A) Glass]

The dental porcelain composition of the present invention comprises (A) glass. The (A) glass is a glass component that melts, and bonds by adhering to the core material by firing. The dental porcelain composition of the present invention may contain only the (A) glass as the glass component. In other words, the dental porcelain composition of the present invention may not contain any glass components other than the (A) glass.

The glass (A) consists of two or more base material glasses and includes at least two base material glasses, that is base material glass (A-1) and base material glass (A-2). The glass (A) may include base material glass (A-3) other than the base material glass (A-1) and the base material glass (A-2). The glass (A) may consist of the base material glass (A-1) and the base material glass (A-2). The dental porcelain composition of the present invention may not contain base material glass (A-3) other than the base material glass (A-1) and the base material glass (A-2). By using only the base material glass (A-1) and the base material glass (A-2) as the glass components in the dental porcelain composition of the present invention, the labor of mixing multiple glasses and the process of manufacturing multiple glasses are not required, therefore it is possible to simplify the manufacturing process of the dental porcelain composition of the present invention.

For the glass (A), D10, D50 and D90 indicating particle diameter satisfy D10 ≤ 5 µm, 5 µm < D50 < 28 µm and 28 µm ≤ D90, may be D10 ≤ 4 µm, 6 µm < D50 < 25 and 32 µm ≤ D90, and, may be D10 ≤ 3 µm, 7 µm < D50 < 20 µm and 36 µm ≤ D90. By setting these numerical ranges, there is a case where the range of an adjustment amount of the (B) organic solvent that make it possible without dripping to use the paste-like or slurry-like dental porcelain composition prepared by mixing the (A) glass with the (B) organic solvent becomes wider and it is possible to suppress the occurrence of a sink mark. In the present invention, "D10", "D50" and "D90" indicate the particle diameters at which the cumulative distribution function of the particle diameter distribution reaches 10%, 50% and 90%, respectively. The average particle diameter of the (A) glass can be determined by measurement methods, for example, using a laser diffraction scattering method, a dynamic light scattering method, a centrifugal sedimentation method, an electrical sensing zone method, a sieving method, a photon correlation spectroscopy method or the like.

For the particle diameter of the (A) glass, a value calculated from (D90-D10)/D50 may be 2.8 or more. Furthermore, it may be 3.0 or more, and may be 3.2 or more. It is possible to recognize that a larger value indicates a broader particle diameter distribution. In this case, there is a case where the range of an adjustment amount of the (B) organic solvent that make it possible without dripping to use the paste-like or slurry-like dental porcelain composition prepared by mixing the (A) glass with the (B) organic solvent becomes wider and it is possible to suppress the occurrence of a sink mark. Further, when the value calculated from (D90-D10)/D50 is less than 2.8, there is a case where the range of an adjustment amount of the (B) organic solvent that make it possible without dripping to use the paste-like or slurry-like dental porcelain composition prepared by mixing the (A) glass with the (B) organic solvent becomes narrower and it may cause the occurrence of a sink mark.

The (A-1) base material glass and the (A-2) base material glass have different particle diameters D50. The particle diameter D50 of the (A-1) base material glass is 2 to 8 µm, and may further be 4 to 6 µm. The particle diameter D50 of the (A-2) base material glass is 25 to 45 µm, and may further be 30 to 40 µm. In the case of using the (A-1) base material glass or the (A-2) base material glass alone, the range of an adjustment amount of the (B) organic solvent that make it possible without dripping to use the paste-like or slurry-like dental porcelain composition prepared by mixing with the (B) organic solvent becomes narrower and a technical work becomes more difficult. When the D50 of the (A-1) base material glass is smaller than 2 µm or larger than 8 µm and when the D50 of the (A-2) base material glass is smaller than 25 µm or larger than 45 µm, there is a case where the range of an adjustment amount of the (B) organic solvent that make it possible without dripping to use the paste-like or slurry-like dental porcelain composition prepared by mixing with the (B) organic solvent becomes narrower and a technical work becomes more difficult.

The mixing ratio of the (A-1) base material glass and the (A-2) base material glass in the (A) glass is not particularly limited, but may be 1:9 to 9:1, 1:4 to 4:1, and 2:3 to 3:2 by mass ratio. By such mixing ratio, it is possible to become wider the adjustment range of the mix of the (B) organic solvent, which makes it possible without dripping to use the paste-like or slurry-like dental porcelain composition prepared by mixing with the (B) organic solvent and to ensure a sufficient single-coat application thickness that can be applied without causing a sink mark. Especially, by using the (A) glass of the present invention, it is possible to prevent the occurrence of a sink mark after firing even when a coating thickness of 0.6 mm or less, 0.4 mm or less, or 0.2 mm or less is applied in one application, without repeatedly perform condensation treatment and water absorption treatment to the dental porcelain composition applied to a core material for example (that is, even when applied dental porcelain composition contains the (B) organic solvent). The "coating thickness" in the present invention means the numerical value of the elevation of the layer surface of the dental porcelain composition after firing with respect to the core material surface before firing as the reference plane. When the mixing ratio of the (A-1) base material glass and the (A-2) base material glass in the (A) glass is not within the range of 1:9 to 9:1 by mass ratio, there is a case where the adjustment range of the mixing of the (B) organic solvent that make it possible without dripping to use the paste-like or slurry-like dental porcelain composition prepared by mixing with the (B) organic solvent becomes narrower and it may cause the occurrence of a sink mark.

The manufacture of the (A-1) base material glass and the (A-2) base material glass can be performed without limitation by the common manufacture device and method of the glass composition held by a person skilled in the art. In one of the common manufacturing method, various inorganic compounds (which may include (C) coloring agents, fluorescent agents (D) and (E) paste-forming stabilizers when these are contained) are compounded so as to obtain a target glass composition to melt at 1300 to 1500 °C in a glass melting furnace. The melt is charged into water to be quenched (quenching) to prepare a glass frit. In order to use the glass frit obtained by this method as the base material glass, it is necessary to powder the glass frit in order to adjust the particle size. Examples of methods for powdering include a method of crushing the glass frit described above with a crusher such as a rotary ball mill, a vibration ball mill, a planetary mill, a jet mill, a bead mill, a roll crusher, a jaw crusher and the like. Further, in order to uniformly adjust the particle size of the base material glass, in addition to the pulverization of the base material glass, classification may be performed using a sieve, an elutriation and the like as necessary. Furthermore, the (C) coloring material, the (D) fluorescent material and the (E) paste stabilizer may be mixed when these are contained.

The (A-1) base material glass and the (A-2) base material glass may have, for example, an elution amount of 50 µg/cm² or less, 35 µg/cm² or less and 20 µg/cm² or less according to "Dentistry-Ceramic materials" of ISO6872:2015/Amd.1:2018. By setting the elution amount to 50 µg/cm² or less, the dental porcelain composition may meet the requirements of the ISO standard.

The (A-1) base material glass and (A-2) base material glass may have, for example, a bending strength of 50 MPa or more, 80 MPa or more and 100 MPa or more according to "Dentistry-Ceramic materials" of ISO6872:2015/Amd.1:2018. By setting the bending strength to 50 MPa or more, the dental porcelain paste may meet the requirements of the ISO standard.

The (A-1) base material glass and (A-2) base material glass may have, for example, a thermal expansion coefficient of 7.0 to 14.0 × 10⁻⁶K⁻¹ and of 7.5 to 11.0 × 10⁻⁶K⁻¹ according to "Dentistry-Ceramic materials" of ISO6872:2015/Amd.1:2018. The dental porcelain composition of the present invention is used, for example, for the purpose of fixing to the upper part of a core material made of oxide ceramics or glass ceramics. Because the thermal expansion coefficient of these core materials are approximately 10.0 to 11.0 × 10⁻⁶K⁻¹, it is possible to suppress the occurrence of cracks during the preparation of dental prosthesis devices, by setting the thermal expansion coefficients of the dental porcelain composition to a range slightly lower the range of core materials.

The glass transition temperatures of the (A-1) base material glass and (A-2) base material glass are not particularly limited, but the glass transition temperature (Tg) according to "Dentistry-Ceramic materials" of ISO6872:2015/Amd.1 :2018 may be set, for example, to 400 to 600 °C and 450 to 550 °C. By using the base material glass having a glass transition temperature of 400 °C to 600°C, it is possible to perform firing the dental porcelain composition within the temperature range of 650 to 850 °C and it is possible to apply to a core material which is prepared from a lithium disilicate base glass ceramics which includes a risk of deformation in firing more than 850 °C.

The (A-1) base material glass and (A-2) base material glass may have, for example, a softening point (Ts) of 450 to 650 °C according to "Dentistry-Ceramic materials" of ISO6872:2015/Amd.1:2018. By using the base material glass having a softening point of 450 °C to 650°C, it is possible to perform firing the dental porcelain composition within the temperature range of 650 to 850 °C and it is possible to apply to a core material which is prepared from a lithium disilicate base glass ceramics which includes a risk of deformation in firing more than 850 °C. In particular, in the present invention, the softening point may be set to 650 °C or less.

For example, a difference in softening point according to ISO 6872:2015/Amd.1:2018 "Dentistry-Ceramic materials" between the base material glass (A-1) and the base material glass (A-2) may be less than 100 °C, preferably 50 °C or less, more preferably 0 °C (combination of the base material glasses having the same softening point). When the difference in softening point is 100 °C or more, there is a case where undissolved portion or glass composition interface may occur within the layer of the dental porcelain composition to generate cloudiness on the surface after firing.

The (A-1) base material glass and the (A-2) base material glass constituting the (A) glass may be mixed without limitation using conventionally known manufacturing device and method. For example, these may be manually mixed by an operator during dental technical work and may be mechanically mixed by grinding and mixing using a rotary ball mill, a vibration ball mill or a planetary mill, mixer mixing and sieve mixing.

Examples of the (A-1) base material glass and the (A-2) base material glass include glass and crystallized glass which contain SiO₂ as a main component (component having the largest content). Such glass may contain Al₂O₃, B₂O₃, ZnO, K₂O, Na₂O, Li₂O, ZrO₂, CaO, MgO and the like, in addition to SiO₂. Further, these may be a glass ceramics containing crystal in glass. By containing crystal, it is expected to adjust the thermal expansion coefficient and to improve mechanical property of the dental porcelain composition. Specific examples include amorphous type potassium aluminosilicate glass, amorphous type potassium borosilicate glass, crystal type potassium aluminosilicate glass, crystal type fluoroapatite glass and crystal type lithium silicate glass. Furthermore, the (A-1) base material glass and the (A-2) base material glass of the present invention may be a glass not containing crystal within the glass.

The glass (A) may contain base material glass (A-3) other than the (A-1) base material glass and the base material glass (A-2). For example, the glass (A) may contain base material glass (A-3) having a particle diameter D50 not satisfying 2 to 8 µm and 25 to 45 µm, as the base material glass other than the (A-1) base material glass and the base material glass (A-2). For example, the compound amount of the (A-3) base material glass may be 0 to 10.0 parts by mass, and 0.1 to 5.0 parts by mass with respect to 100.0 parts by mass of the total amount of the (A) glass. When the (A) glass contains the (A-3) base material glass, the particle diameters D10, D50 and D90 of the (A) glass are calculated including the (A-3) base material glass. Furthermore, the (A) glass may not contain (A-3) base material glass having a particle diameter D50 not satisfying 2 to 8 µm or 25 to 45 µm as a base material glass other than the (A-1) base material glass and the (A-2) base material glass. By this configuration, it is possible to reduce the risk that the adjustment range of the mixing of the (B) organic solvent that make it possible without dripping to use the paste-like or slurry-like dental porcelain composition prepared by mixing with the (B) organic solvent becomes narrower and the risk of occurrence of a sink mark. For example, the (A) glass may contain only the (A-1) base material glass and the (A-2) base material glass.

### [(B) Organic solvent]

The dental porcelain composition of the present invention comprises (B) organic solvent. The (B) organic solvent is used to mix with the (A) glass to form a paste. The organic solvent used in the present invention can be fired simultaneously with the base material glass without removal operations involving condensation and water absorption. Furthermore, the organic solvent used in the present invention disappears by incineration after firing the dental ceramic composition. Therefore, it is a component that does not ultimately remain in the dental prosthesis device.

As the (B) organic solvent used in the present invention, an organic solvent having a boiling point of 100 to 300 °C is used. Furthermore, in the present invention, an organic solvent having a boiling point of 270 °C or less may be used, and an organic solvent having a boiling point of 250 °C or less may be used. When the boiling point of the organic solvent is less than 100 °C, the organic solvent volatilizes even at room temperature to cause dry and deterioration operability during technical work. When the boiling point of the organic solvent exceeds 300 °C, it cause unburnt residue during firing, causing carbonization and bubbles.

The (B) organic solvent used in the present invention is not particularly limited as long as it has a boiling point of 100 to 300 °C, but specific examples include ester base solvents such as dimethyl phthalate and diethyl phthalate; polyhydric alcohol base solvents such as 1,2-butanediol, 1,3-butanediol, 1,4-butanediol, 1,5-pentanediol, glycerin, diethylene glycol, triethylene glycol, propylene glycol and dipropylene glycol; polyhydric alcohol monoether base solvents such as ethylene glycol monomethyl ether, ethylene glycol monoethyl ether, propylene glycol monomethyl ether, propylene glycol monoethyl ether, ethylene glycol monobutyl ether, diethylene glycol monomethyl ether, diethylene glycol monoethyl ether, propylene glycol monopropyl ether, tripropylene glycol monomethyl ether, diethylene glycol monobutyl ether and triethylene glycol monomethyl ether; and aromatic alcohol solvents such as 2-phenoxy ethanol and benzyl alcohol. Among these organic solvents, polyhydric alcohol base solvents, polyhydric alcohol monoether base solvents and aromatic alcohol solvents may be used and 1,3-butanediol, propylene glycol and 2-phenoxy ethanol may be used. These organic solvents (B) can be used not only singly but also in appropriate combinations of two or more. When two or more kinds of organic solvents are used in combination, the total value obtained by multiplying the boiling points of the respective organic solvents to be used by the addition ratio is used as the boiling point of the (B) organic solvent.

The (B) organic solvent used in the present invention may consist only of an organic solvent having a boiling point of 250 °C or less, and the dental porcelain composition of the present invention may not contain an organic solvent having a boiling point higher than 250 °C. In the case of containing an organic solvent having a boiling point higher than 250 °C, there is a case where it remains as unburned residue during firing to cause carbonization or a bubble.

The (B) organic solvent used in the present invention may be an alcohol having a hydroxy group. The reason for this is to improve the compatibility with the base material glass having a hydroxyl group to improve the applicability.

When the (B) organic solvent is a component having solubility in water, the dental porcelain composition of the present invention may contain water. In this case, the organic solvent may be an alcohol containing a hydroxyl group. When the dental porcelain composition of the present invention contains water, the amount of water may be 30 parts by mass or less, with respect to 100 parts by mass of the total of water and the organic solvent.

In the present invention, the compounding amount of the (B) organic solvent may be 10 to 200 parts by mass with respect to 100 parts by mass of the glass (A), and may be 20 to 150 parts by mass. Particularly, in the present invention, since it is possible to use while drip property is suppressed, the compounding amount of the (B) organic solvent may be 20 to 80 parts by mass with respect to 100 parts by mass of the (A) glass. When the compounding amount of the organic solvent is too small, it may be difficult to form a paste. When the compounding amount of the organic solvent is too large, there is a tendency that the paste easily drip, especially in the case of applying thick layers.

### [Other components]

The (A-1) base material glass and the (A-2) base material glass in the present invention may contain (C) coloring material, (D) fluorescent material and/or (E) paste stabilizer. Furthermore, the dental porcelain composition of the present invention may contain (C) coloring material, (D) fluorescent material and/or (E) paste stabilizer in a form not contained in the (A-1) base material glass and the (A-2) base material glass (that is, as separate component). The (C) coloring material and/or the (D) fluorescent material are components imparting color tone and fluorescence to the dental prosthesis device. The (E) paste stabilizer may impart to the paste prepared by mixing the (A) glass and the (B) organic solvent, a sedimentation-inhibiting effect that prolongs the paste state or an effect that facilitates to back into the paste state of the solidified material after sedimentation.

The coloring material (C) is, for example, an inorganic material, and those commonly used in the dental materials can be used. Specific examples include a coloring material prepared by mixing a plurality of metal oxides such as SiO₂, Al₂O₃, CaO, TiO₂, SnO, Cr₂O₃, MnO, Sb₂O₃, V₂O₅, ZnO, Fe₂O₃, W₂O₃, Co₂O₃ and ZrO₂, and firing. The compounding amount of the (C) coloring agent may be 0.0 to 40.0 parts by mass with respect to 100.0 parts by mass of the total of the (A) glass, and the (C) coloring material, the (D) fluorescent material and the (E) paste stabilizer when these are contained. When the content exceeds 40 parts by mass, there is a case where the baking of the glass layer decrease and the bond by adhesion to the core material is insufficient.

The (D) fluorescent material is, for example, an inorganic material, and those commonly used in the dental materials can be used. Specific examples include a coloring material prepared by mixing a plurality of metal oxides such as SiO₂, Al₂O₃, CaO, TiO₂, SnO, Cr₂O₃, MnO, Sb₂O₃, V₂O₅, ZnO, Fe₂O₃, W₂O₃, Co₂O₃ and ZrO₂, and firing. The compounding amount thereof may be 0.0 to 40 parts by mass with respect to 100.0 parts by mass of the total of the (A) glass, and the (C) coloring material, the (D) fluorescent material and the (E) paste stabilizer when these are contained, and may be 40 parts by mass or less. When the content exceeds 40 parts by mass, there is a case where the baking of the glass layer decreases and the bond by adhering to the core material is insufficient.

The paste stabilizer (E) is, for example, an inorganic material or an inorganic salt, and those commonly used in the dental materials can be used. Specific examples of the inorganic material include fine-particle silica or fine-particle titanium having an average primary particle diameter of 1 to 50 nm, 5 to 45 nm, or 7 to 40 nm, and these may be used alone or in an appropriate combination of two or more kinds. Specific examples of the inorganic salt include a chloride, a nitrate, a sulfate, a carbonate and/or a hydrate thereof , which includes an element selected from Al, Zn, K, Na, Li, Ca and Mg. Further specific examples include aluminum chloride, aluminum nitrate, zinc chloride, zinc nitrate, potassium chloride, potassium sulfate, sodium chloride, sodium sulfate, lithium chloride, lithium nitrate, calcium chloride, calcium nitrate, calcium sulfate, magnesium nitrate, magnesium sulfate, and/or hydrates thereof and these may be used alone or in an appropriate combination of two or more kinds. The compounding amount thereof may be 0.0 to 10 parts by mass with respect to 100.0 parts by mass of the total of the (A) glass, and the (C) coloring material, the (D) fluorescent material and the (E) paste stabilizer when these are contained, and may be 10 parts by mass or less. When the content exceeds 10 parts by mass, there is a case where the baking of the glass layer decreases and the bond by adhering to the core material is insufficient.

The dental porcelain composition of the present invention contains essentially no organic polymeric material. The "organic polymeric material" in the present invention means an organic component having a molecular weight of 200 or more that are separately added for purposes other than surface treatment. Such organic component is poorly volatile and may remain within the porcelain layer during the firing stage. The dental porcelain composition of the present invention may not contain a solvent other than the (B) organic solvent. The "contains essentially no" in the present invention means less than 0.1 parts by weight with respect to 100 parts by weight of the total dental porcelain composition. The content of the organic polymeric material contained in the dental porcelain composition of the present invention may be 0.00 parts by weight.

### [Manufacturing method of dental porcelain composition]

The dental porcelain composition of the present invention can be manufactured without limitation by the common manufacturing method of the paste composition held by a person skilled in the art. In one of the common preparation method, the paste is prepared by compounding a base material glass, an organic solvent, a coloring material, a fluorescent material and a paste stabilizer so that a target paste composition is obtained, and mixing with a device comprising a stirring and/or defoaming function. It is possible to perform the mixing by a known kneading method using a rotary mixer equipped with stirring blades, a rotation-revolution mixer, a crusher, a roll mill, a ball mill, a kneader and the like, and these methods can be used alone or in combination.

The dental porcelain composition of the present invention can be manufactured by mixing the (A) glass (base material glass) and the organic solvent on a kneading by a dental technician in a dental laboratory so as to prepare the desired paste composition without mechanical processing. For mixing on the kneading plate, common dental laboratory tools such as spatulas or glass rods can be used. By using this method, for example, it is possible to prepare a paste by kneading the (B) organic solvent into a powder mixture of the (A-1) base material glass and the (A-2) base material glass that has been pre-mixed. Alternatively, it is possible to prepare a paste by kneading the (A-1) base material glass with the (B) organic solvent to prepare pre-paste, and then mixing the (A-2) base material glass into this pre-paste. In particular, by adopting the method of mixing the (A-2) base material glass having larger-particle size at a later stage into the paste prepared by kneading the (A-1) base material glass with the (B) organic solvent, it is possible to minimize the viscosity change behavior, and adjustment to the desired viscosity becomes easier.

### [Dental porcelain composition kit]

The components constituting the dental porcelain composition of the present invention may be combined to form a dental porcelain composition kit. Specific dental porcelain composition kit may comprise a glass prepared by pre-mixed with the (A-1) base material glass and the (A-2) base material glass, and an (B) organic solvent. Furthermore, the dental porcelain composition kit may comprise the (A-1) base material glass, the (A-2) base material glass and the (B) organic solvent. Furthermore, the dental porcelain composition kit may comprise the (A-1) base material glass, the (A-2) base material glass, the (B) organic solvent and an organic solvent (B') having a composition different from that of the (B) organic solvent. Furthermore, the dental porcelain composition kit may comprise a mixed glass prepared by pre-mixing the (A-1) base material glass and the (A-2) base material glass, the (B) organic solvent and an organic solvent (B') having a composition different from that of the (B) organic solvent. Since the viscosity characteristics of the paste (dental porcelain composition) prepared by mixing with the glass differ depending on the composition of the organic solvent, it is possible to provide a dental porcelain composition kit that allows dental technicians to easily select the optimal viscosity by comprising two kinds of organic solvents with different compositions.

### [Examples]

Hereinafter, the present invention will be described in detail with reference to Examples and Comparative Examples. However, the present invention is not limited to these Examples.

The manufacturing method of the glass and the base material glass, the measuring method of the particle diameter, the measuring method of presence or absence of crystal and the measuring method of various physical properties and the various evaluation methods of the dental porcelain composition in Examples and Comparative Examples are described below.

### [Base material glass]

Glasses G1 to G5 containing SiO₂, Al₂O₃, K₂O, Na₂O and other components (B₂O₃, Li₂O, CaO, ZnO, F, MgO or the like) in the ratio shown in Table 1 were prepared by a melting method, and then crushed using various general crushers to prepare base material glasses (A-1-1) to (A-3-1). As base material glass (A-1-10), AS (A shade tone) of Vintage Art Universal (manufactured by SHOFU INC.), which is a commercially available coloring material for dental ceramics, was used. Base material glass (a-1-10) contains, in addition to the base material glass components, (e) coloring material selected from an inorganic material and (f) fluorescent material selected from an inorganic material, and has 5 µm of the particle diameter D50.

Details of each glass are as follows.

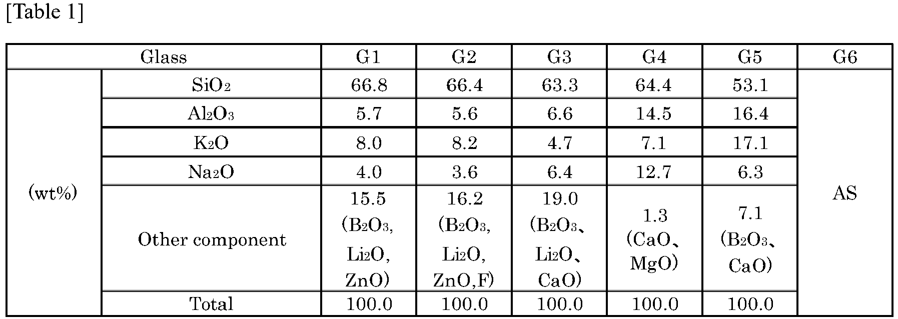

<Manufacturing of base material glass (A-1-1)>
   Glass G1 was ground using a bead mill crusher until D50 was 2.0 µm.
<Manufacturing of base material glass (A-1-2)>
   Glass G1 was ground using a vibration bead mill crusher until D50 was 4.0 µm.
<Manufacturing of base material glass (A-1-3)>
   Glass G1 was ground using a vibration bead mill crusher until D50 was 5.0 µm.
<Manufacturing of base material glass (A-1-4)>
   Glass G1 was ground using a vibration bead mill crusher until D50 was 6.0 µm.
<Manufacturing of base material glass (A-1-5)>
   Glass G1 was ground using a vibration bead mill crusher until D50 was 8.0 µm.
<Manufacturing of base material glass (A-1-6)>
   Glass G2 was ground using a vibration bead mill crusher until D50 was 5.0 µm.
<Manufacturing of base material glass (A-1-7)>
   Glass G3 was ground using a vibration bead mill crusher until D50 was 6.0 µm.
<Manufacturing of base material glass (A-1-8)>
   Glass G4 was subjected to a heat treatment step to precipitate leucite crystals, and then ground using a jet mill crusher until D50 became 5.0 µm.
<Manufacturing of base material glass (A-1-9)>
   Glass G5 was subjected to a heat treatment step to precipitate leucite crystals, and then ground using a jet mill crusher until D50 became 5.0 µm.
<Manufacturing of base material glass (A-2-1)>
   Glass G1 was ground using a ball mill crusher until D50 was 25.0 µm.
<Manufacturing of base material glass (A-2-2)>
   Glass G1 was ground using a ball mill crusher until D50 was 30.0 µm.
<Manufacturing of base material glass (A-2-3)>
   Glass G1 was ground using a ball mill crusher until D50 was 35.0 µm.
<Manufacturing of base material glass (A-2-4)>
   Glass G1 was ground using a ball mill crusher until D50 was 40.0 µm.
<Manufacturing of base material glass (A-2-5)>
   Glass G1 was ground using a ball mill crusher until D50 was 45.0 µm.
<Manufacturing of base material glass (A-3-1)>
   Glass G1 was ground using a jet mill crusher until D50 was 20.0 µm.

### [Measuring method of particle diameter D50 of base material glass]

The average particle diameter D50 of the base material glass can be determined by a measurement using a laser diffraction scattering method, a dynamic light scattering method, a centrifugal sedimentation method, an electrical sensing zone method, a sieving method, a photon correlation spectroscopy method or the like. The particle diameters of the glass powders of Examples and Comparative Examples were measured by a laser diffraction scattering method. The particle diameter of the base material glass in Examples and Comparative Examples was measured using a laser diffraction/ scattering method. Specifically, the measurement was performed using laser diffraction type particle size distribution measuring device Microtrac MT-3000II (manufactured by MicrotracBEL Corp.), under the following conditions: dispersion medium: water, sample refractive index: 1.51, particle shape: amorphous.

### [Measuring method of presence or absence of crystal]

The presence or absence of crystals in the base material glass can be confirmed by measurement using an X-ray diffraction device. Specifically, the measurement was performed using the X-ray diffraction device SmaertLab SE (manufactured by Rigaku Corp.) at a scanning range of 10 to 70° and a scanning speed of 2.0°/min.

### [Measuring method of glass transition temperature, softening point and thermal expansion coefficient of base material glass]

Each base material glass was kneaded with distilled water to prepare a kneaded material. The kneaded material was filled in a stick type mold made of silicon (6 × 6 × 25 mm) and was subjected to condensation and water absorption repeatedly to prepare a molded body. The molded body was taken out from the silicon mold and was fired twice including one time vacuum firing and one time atmospheric firing by the dental technique porcelain furnace "Esthemat Slim" (manufactured by SHOFU INC.). Sample was prepared by polishing both ends of the prepared twice fired product to prepare parallel faces and adjusting the size to 5 × 5 × 20 mm. Glass transition temperature, softening point and thermal expansion coefficient of the sample were measured by the thermal expansion meter "TM8140C" (manufactured by Rigaku Corp.) according to the procedure of ISO6872:2015 /Amd.1:2018 "Dentistry-Ceramic materials".

### [Measuring method of elution amount of base material glass]

Each base material glass was kneaded with distilled water to prepare a kneaded material. The kneaded material was filled in a disc type mold made of silicon (φ12 mm x 2 mm) and was subjected to condensation and water absorption repeatedly to prepare a molded body. Ten fired products were prepared by taking out the molded body from the silicon mold and vacuum firing by the dental technique porcelain furnace "Esthemat Slim" (manufactured by SHOFU INC.). After surface polish of both surfaces of the fired products, second firing (atmospheric firing) was performed. The test specimen was tested according to the procedure of ISO6872:2015 /Amd.1:2018 "Dentistry-Ceramic materials".

### [Measuring method of bending strength of base material glass]

Each base material glass was kneaded with distilled water to prepare a kneaded material. The kneaded material was filled in a stick type mold made of silicon (3 × 6 × 25 mm) and was subjected to condensation and water absorption repeatedly to prepare a molded body. Ten fired products were prepared by taking out the molded body from the silicon mold and vacuum firing by the dental technique porcelain furnace "Esthemat Slim" (manufactured by SHOFU INC.). The entire surface of the prepared fired product was polished to make parallel surfaces, and the sample adjusted to a size of 1.2 x 4 x 20 mm was used as a test specimen. The bending strength was measured using a universal testing machine (manufactured by Shimadzu Corporation) according to the procedure of ISO6872:2015 /Amd.1:2018 "Dentistry-Ceramic materials".

### [Evaluation 1: Measurement of D10, D50 and D90 of (A) glass, and Calculation of (D90-D10)/D50]

The base material glass (A-1-1) to (A-3-1) was sieve mixed to prepare (A) glass. The particle diameters D10, D50 and D90 of the (A) glass were measured using a laser diffraction type particle size distribution measuring device Microtrac MT-3000II (manufactured by MicrotracBEL Corp.), under the following conditions: dispersion medium: water, sample refractive index: 1.51, particle shape: amorphous. Furthermore, by using the D10, D50 and D90 values obtained for each glass, the numerical value was calculated using the formula '(D90-D10)/D50'. The evaluation criteria for this value are shown below.
3.2 or higher: Particle diameter distribution is sufficiently broad.
3.0 or higher and less than 3.2: Particle diameter distribution is broad.
2.8 or higher and less than 3.0: Particle diameter distribution is applicable broad.
Less than 2.8: Particle diameter distribution is narrow.

### [Evaluation 2: Drip property]

The glass, the solvent and the equipment used for evaluation were placed in a thermostatic chamber at 23 °C 1 hour prior to evaluation. The glass and the solvent used in Examples and Comparative Examples shown in Tables 3 to 5 were mixed to prepare a paste. At this time, the solvent was weighed to be 0.70 parts by mass, 0.65 parts by mass, 0.60 parts by mass, 0.55 parts by mass, 0.50 parts by mass, 0.45 parts by mass, 0.40 parts by mass, 0.35 parts by mass and 0.30 parts by mass with respect to 1.0 parts by mass of the glass, and mixed. By this, nine mixtures were prepared for each type of the glass. Using samples (compositions) where the mixture formed a uniform paste, the following tests were conducted.

A sample weighed at 0.1±0.01 g was gently placed at the center of a glass plate (70 mm × 70 mm × 1 mm). The sample was left to stand in this state for 30 seconds. The diameter of the spread circle of the paste after standing (value before dripping) was recorded. The glass plate placed with the sample was then rotated 90° so that the 70 mm × 70 mm surface was vertical and was left to stand for 30 seconds. The length of the paste that dripped after standing (value after dripping) was recorded. The difference between the value after dripping and the value before dripping was calculated as the "drip value" for the sample. The calculated "drip value" for each sample was evaluated as the "drip property" of the dental porcelain composition corresponding to the sample in the respective Examples and Comparative Examples. The evaluation criteria for "drip property" are as follows.
AA: The number of samples where a paste-like state was maintained and the drip value was 0 to 10 mm was five or more. It was recognized that the adjustment range of the solvent ((B) organic solvent) that makes the porcelain composition that is less likely to drip was wide.
A: The number of samples where a paste-like state was maintained and the drip value was 0 to 10 mm was four. It was recognized that the adjustment range of the solvent ((B) organic solvent) that makes the porcelain composition that is less likely to drip was somewhat wide.
B: The number of samples where a paste-like state was maintained and the drip value was 0 to 10 mm was three. It was recognized that the adjustment range of the solvent ((B) organic solvent) that makes the porcelain composition that is less likely to drip was slightly wide.
C: The number of samples where a paste-like state was maintained and the drip value was 0 to 10 mm was two or less. It was recognized that the adjustment range of the solvent ((B) organic solvent) that makes the porcelain composition that is less likely to drip was equivalent to or less than that of the conventional porcelain composition.

### [Evaluation 3: Sink mark]

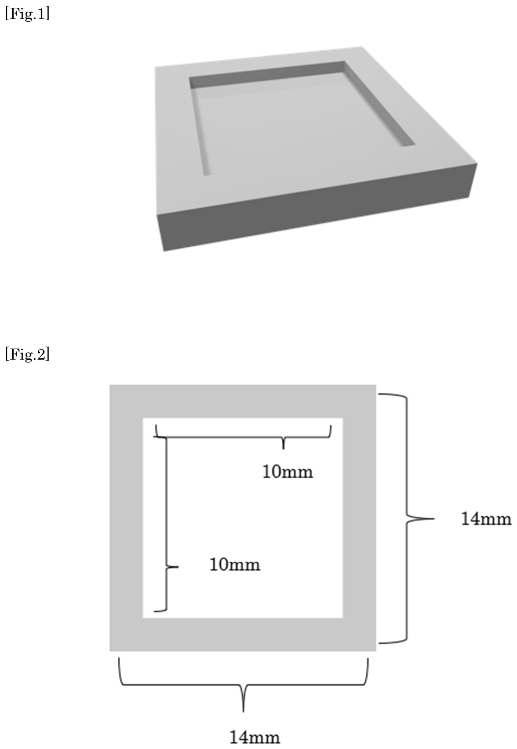

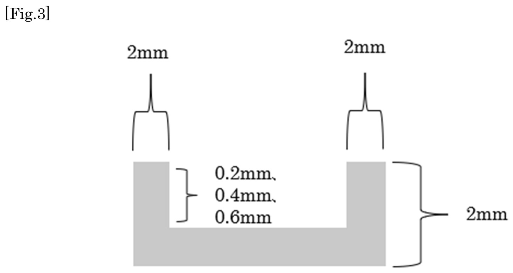

For each porcelain composition, a porcelain composition corresponding to the sample having the highest solvent-to-glass ratio among the samples where a paste-like state was maintained and the drip value was 0 to 10 mm in Evaluation 2 was prepared. Using "SHOFU DISK ZR-SS COLORED, Color Tone: Peach Light" manufactured by SHOFU INC., zirconia base materials having the shapes shown in Figures 1 to 3 were prepared. The porcelain composition was applied to the 10.0 mm × 10.0 mm recessed surface of the base material using a dental technician's brush until the porcelain composition can be leveled. Using the dental technique porcelain furnace "Esthemat Slim II" (manufactured by SHOFU INC.), after drying outside the furnace for 7 minutes, vacuum firing was performed at 750 °C. For each porcelain composition, tests were conducted using three types of base materials with different depression depths (0.2 mm, 0.4 mm and 0.6 mm). The presence or absence of sink mark, such as cracking in the applied layer and peeling from the depression walls of the base material after firing, was visually evaluated. The evaluation criteria are as follows.
AA: Sink mark was not observed on three kinds of the base material. It was recognized that sink mark did not occur when firing the porcelain composition at coating thicknesses up to 0.6 mm.
A: Sink mark was not observed on two kinds of the base material (depression depths 0.2 mm and 0.4mm). It was recognized that sink mark did not occur when firing the porcelain composition at coating thicknesses up to 0.4 mm.
B: Sink mark was not observed on one kind of base material (depression depth 0.2 mm). It was recognized that sink mark did not occur when firing the porcelain composition at coating thicknesses up to 0.2 mm.
C: Sink mark was observed on three kinds of the base material. It was recognized that sink mark occurs when firing the porcelain composition at coating thicknesses up to 0.2 mm.

### [Evaluation 4: Cloudiness of fired product]

The porcelain layer of the fired products prepared in Evaluation 3 was visually evaluated. The evaluation criteria are as follows.
AA: Cloudiness was not observed on three kinds of the base material. It was recognized that cloudiness did not occur when firing the porcelain composition at coating thicknesses up to 0.6 mm.
A: Cloudiness was not observed on two kinds of the base material (depression depths 0.2 mm and 0.4mm). It was recognized that cloudiness did not occur when firing the porcelain composition at coating thicknesses up to 0.4 mm.
B: Cloudiness was not observed on one kind of base material (depression depth 0.2 mm). It was recognized that cloudiness did not occur when firing the porcelain composition at coating thicknesses up to 0.2 mm.
C: Cloudiness was observed on three kinds of the base material. It was recognized that cloudiness occurs when firing the porcelain composition at coating thicknesses up to 0.2 mm.

### [Evaluation 5: Black discoloration of fired product]

The porcelain layer of the fired products prepared in Evaluation 3 was visually evaluated. The evaluation criteria are as follows.
AA: Black discoloration was not observed on three kinds of the base material. It was recognized that black discoloration did not occur when firing the porcelain composition at coating thicknesses up to 0.6 mm.
A: Black discoloration was not observed on two kinds of the base material (depression depths 0.2 mm and 0.4mm). It was recognized that black discoloration did not occur when firing the porcelain composition at coating thicknesses up to 0.4 mm.
B: Black discoloration was not observed on one kind of base material (depression depth 0.2 mm). It was recognized that black discoloration did not occur when firing the porcelain composition at coating thicknesses up to 0.2 mm.
C: Black discoloration was observed on three kinds of the base material. It was recognized that black discoloration occurs when firing the porcelain composition at coating thicknesses up to 0.2 mm.

Table 2 shows the test results for each base material glass (A-1-1) to (A-3-1).

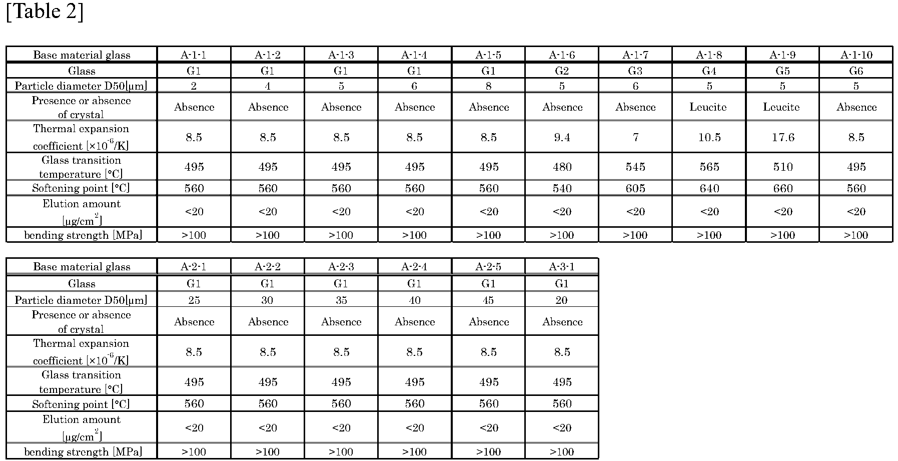

Details of each Example and Comparative Example are shown below.

### <Examples 1-20 and Comparative Examples 2, 3>

The (A-1) base material glass and the (A-2) base material glass were sieve-mixed to form the (A) glass. The (A) glass was kneaded on a glass kneading plate using 2-phenoxyethanol (boiling point: 247 °C, molecular weight: 138.16) as the (B) organic solvent to prepare a paste-like porcelain composition.

### <Example 21>

The (A-1) base material glass was kneaded on a glass kneading plate using 2-phenoxyethanol (boiling point: 247 °C, molecular weight: 138.16) as the (B) organic solvent to form a paste. The remaining (A-2) base material glass was then further mixed and kneaded to prepare a paste-like porcelain composition.

### <Example 22>

The (A-1) base material glass and the (A-2) base material glass were sieve-mixed to form the (A) glass. Using a solvent (calculated boiling point: 227 °C) prepared by mixing 1,3-butanediol (boiling point: 207 °C, molecular weight: 90.12) and 2-phenoxyethanol (boiling point: 247 °C, molecular weight: 138.16) in a 1:1 mass ratio (calculated boiling point: 227 °C) as (B) organic solvent, the (A) glass was kneaded on a glass kneading plate to prepare a paste-like porcelain composition.

### <Example 23>

A porcelain composition is prepared by using the (A-1) base material glass and the (A-2) base material glass, a mixture of fine-particle silica R974 (average primary particle diameter 12 nm, manufactured by NIPPON AEROSIL CO., LTD.) and calcium nitrate as the (E)paste stabilizer, 1,3-butanediol (boiling point: 207 °C, molecular weight: 90.12) as the (B) organic solvent, and water. The water content was 25 parts by mass with respect to 100 parts by mass of the total of the water and the organic solvent. This porcelain composition was kneaded using a rotating-revolving mixer to form a paste-like porcelain composition.

### <Example 24>

The (A-1) base material glass and the (A-2) base material glass were sieve-mixed to form the (A) glass. Using a solvent (calculated boiling point: 262°C) prepared by mixing 2-phenoxyethanol (boiling point: 247 °C, molecular weight: 138.16) and glycerin (boiling point: 290 °C, molecular weight: 92.09) in 2:1 mass ratio as (B) organic solvent, the (A) glass was kneaded on a glass kneading plate to prepare a paste-like porcelain composition.

### <Example 25>

The (A-1) base material glass and the (A-2) base material glass were sieve-mixed to form the (A) glass. The (A) glass was kneaded on a glass kneading plate using glycerin (boiling point: 290 °C, molecular weight: 92.09) as the (B) organic solvent to prepare a paste-like porcelain composition.

### <Example 26>

The (A-1) base material glass, the (A-2) base material glass, and the (A-3) base material glass were sieve-mixed to form the (A) glass. Using 2-phenoxyethanol (boiling point: 247 °C, molecular weight: 138.16) as the (B) organic solvent, the (A) glass was kneaded on a glass kneading plate to prepare a paste-like porcelain composition.

### <Comparative Examples 1 and 4>

The glass was prepared using only either the (A-1) base material glass or the (A-2) base material glass. Using 2-phenoxyethanol (boiling point: 247 °C, molecular weight: 138.16) as the (B) organic solvent, the glass was kneaded on a glass kneading plate to prepare a paste-like porcelain composition.

### <Comparative Example 5>

The (A-1) base material glass and the (A-2) base material glass were sieve-mixed to form the (A) glass. The (A) glass was kneaded on a glass kneading plate using benzyl benzoate (boiling point: 323 °C, molecular weight: 212.25) as the (B) organic solvent to prepare a paste-like porcelain composition.

### <Comparative Example 6>

The (A-1) base material glass and the (A-3-1) base material glass were sieve-mixed to form the (A) glass. The (A) glass was kneaded on a glass kneading plate using 2-phenoxyethanol (boiling point: 247 °C, molecular weight: 138.16) as the (B) organic solvent to prepare a paste-like porcelain composition.

### <Comparative Example 7>

The (A-2) base material glass and the (A-3-1) base material glass were sieve-mixed to form the (A) glass. The (A) glass was kneaded on a glass kneading plate using 2-phenoxyethanol (boiling point: 247 °C, molecular weight: 138.16) as the (B) organic solvent to prepare a paste-like porcelain composition.

The compositions of the porcelain compositions prepared in Examples and Comparative Examples and various test results are shown in Tables 3 to 5. Regarding the (B) organic solvent, the check mark indicates the type of the used organic solvent, and its amount is as described in the above "Evaluation 3".

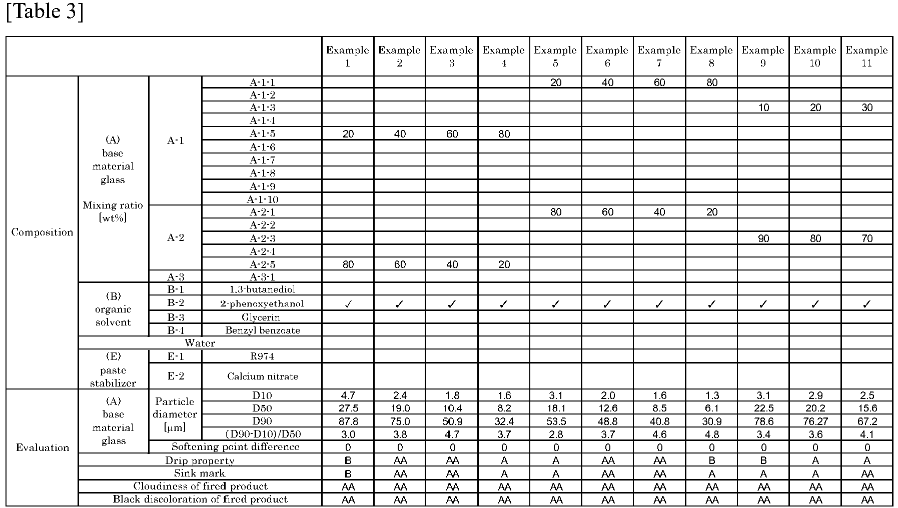

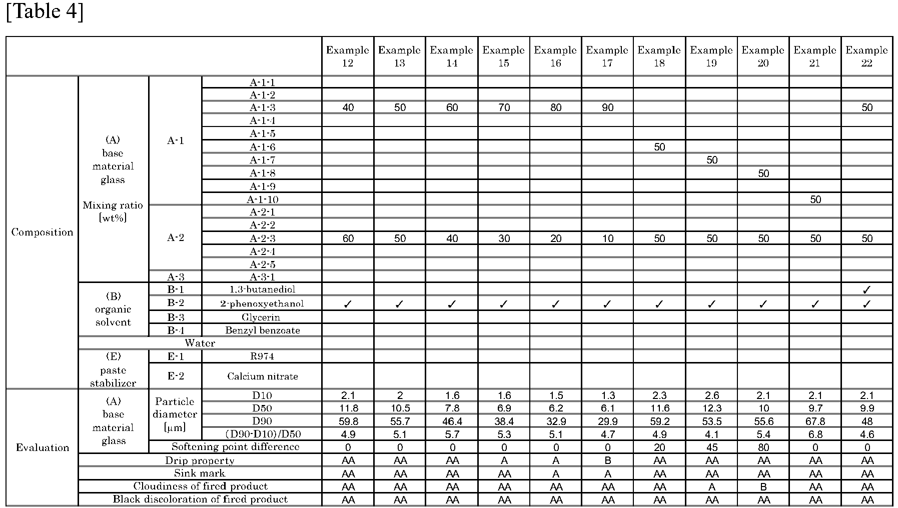

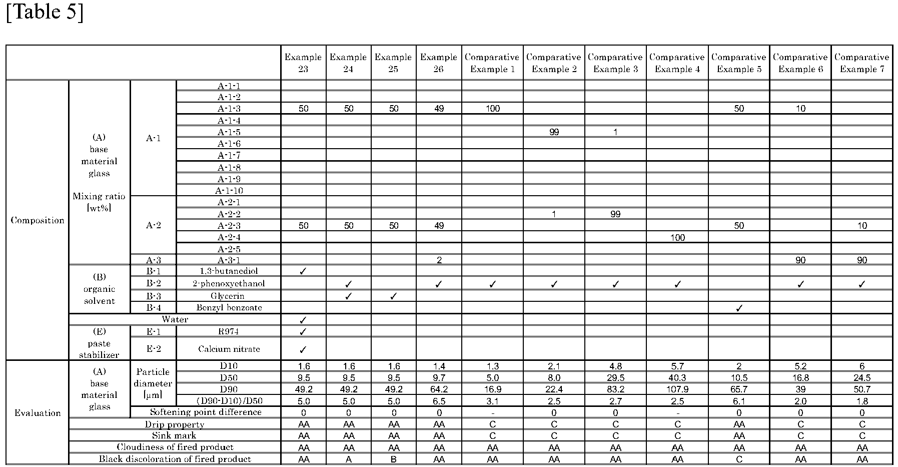

In Examples 1 to 26, the good results were exhibited in the tests of the drip property and the sink mark, and black discoloration and cloudiness in the fired product were not observed.

On the other hand, there were following problems in Comparative Examples.

In Comparative Examples 1 to 4, because only a single type of the base material glass was used or the mixing state of the base material glasses was inappropriate, the particle diameters D10, D50 and D90 of the glass (A) were not appropriate values, and drip property decreased and sink mark occurred.

In Comparative Example 5, because of using benzyl benzoate as the organic solvent, the black discoloration occurred on the surface after firing.

In Comparative Examples 6 and 7, because of using only one of the (A-1) base material glass or the (A-2) base material glass, the particle diameters D10, D50 and D90 of the glass (A) were not appropriate values, and drip property decreased and sink mark occurred.

Based on the above results, the dental porcelain composition provided by the present disclosure is characterized by not requiring the skilled condensation operation, and having excellent characteristics in dripping property, sink mark suppression and aesthetic property after firing to significantly improve the problem in the prior art.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The dental porcelain paste provided by the present invention is characterized by not requiring the skilled condensation operation, and having excellent characteristics in dripping property, sink mark suppression and aesthetic property after firing. Therefore, it can be applied to various dental crown restorations in restorative treatment in the dental field.

## Claims

1. A dental porcelain composition comprising (A) glass and (B) organic solvent having a boiling point of 100 to 300 °C, wherein
the (A) glass includes (A-1) base material glass having a particle diameter D50 of 2 to 8 µm and (A-2) base material glass having a particle diameter D50 of 25 to 45 µm, and
a particle diameter of the glass (A) satisfies D10 ≤ 5 µm, 5 µm < D50 < 28 µm, and 28 µm ≤ D90.

2. The dental porcelain composition according to claim 1, wherein
a mixing ratio of the (A-1) base material glass and the (A-2) base material glass is 9:1 to 1:9 by mass ratio.

3. The dental porcelain composition according to claim 1 or 2, wherein
a value of (D90-D10)/D50 for the particle diameter of the glass (A) is 2.8 or more.

4. The dental porcelain composition according to any one of claims 1 to 3, wherein
softening points according to ISO 6872:2015/Amd.1:2018 "Dentistry-Ceramic materials" of the (A-1) base material glass and the (A-2) base material glass are 650 °C or less.

5. The dental porcelain composition according to any one of claims 1 to 4, wherein
a difference in softening point according to ISO 6872:2015/Amd.1:2018 "Dentistry-Ceramic materials" between the base material glass (A-1) and the base material glass (A-2) is less than 100 °C.

6. The dental porcelain composition according to any one of claims 1 to 5, wherein
a compounding amount of the (B) organic solvent is 10 to 200 parts by mass with respect to 100 parts by mass of the (A) glass.

7. The dental porcelain composition according to any one of claims 1 to 6, wherein
the (B) organic solvent consists of an organic solvent having a boiling point of 250 °C or less.

8. The dental porcelain composition according to any one of claims 1 to 7, wherein
the (A) glass consists of the (A-1) base material glass and the (A-2) base material glass.

9. A manufacturing method of a dental porcelain composition, wherein
a dental porcelain composition is manufactured by mixing (A-1) base material glass having a particle diameter D50 of 2 to 8 µm, (A-2) base material glass having a particle diameter D50 of 25 to 45 µm, and (B) organic solvent having a boiling point of 100 to 300 °C, and
a particle diameter of the glass (A) including the (A-1) base material glass and the (A-2) base material glass satisfies D10 ≤ 5 µm, 5 µm < D50 < 28 µm, and 28 µm ≤ D90.

10. The manufacturing method of a dental porcelain composition according to claim 9, comprising
a step of mixing the (A-1) base material glass and the (A-2) base material glass to manufacture a powder, and
a step of kneading the (B) organic solvent with the powder.

11. The manufacturing method of a dental porcelain composition according to claim 9 or 10, comprising
a step of kneading the (A-1) base material glass and the (B) organic solvent to manufacture a paste, and
a step of mixing the (A-2) base material glass with the paste.

12. A manufacturing method of a dental prosthesis device, comprising
a step of applying the dental porcelain composition according to claim 1 to a core material, and
a step of firing the applied dental porcelain composition.

13. The manufacturing method of a dental prosthesis device according to claim 12, wherein the core material is made of ceramics, glass ceramics or glass.

14. The manufacturing method of a dental prosthesis device according to claim 12 or 13, wherein in the step of firing the applied dental porcelain composition, the dental ceramic composition contains the (B) organic solvent.

15. A kit of dental porcelain composition for manufacturing a dental porcelain composition, comprising
(A-1) base material glass having a particle diameter D50 of 2 to 8 µm,
(A-2) base material glass having a particle diameter D50 of 25 to 45 µm, and
(B) organic solvent having a boiling point of 100 to 300 °C.
